# EUROPÄISCHE PATENTSCHRIFT

(11) **EP 1 035 917 B1**
(45) Veröffentlichungstag und Bekanntmachung des Hinweises auf die Patenterteilung: **06.03.2002**
(21) Anmeldenummer: 98961246.0
(22) Anmeldetag: 01.12.1998
(51) Int. Cl.: B01J 31/22, C07C 33/042, C07C 209/60

(54) **VERFAHREN ZUR HERSTELLUNG EINES KATALYSATORS UND NACH DIESEM VERFAHREN ERHÄLTLICHE KATALYSATOREN**
METHOD FOR PREPARING A CATALYST AND CATALYSTS PREPARED ACCORDINGLY
PROCEDE DE PREPARATION D'UN CATALYSEUR ET CATALYSEURS OBTENUS A L'AIDE DUDIT PROCEDE

(30) Priorität: 02.12.1997 DE 19753458
(43) Veröffentlichungstag der Anmeldung: 20.09.2000
(73) Patentinhaber: BASF AKTIENGESELLSCHAFT, 67056 Ludwigshafen (DE)
(72) Erfinder: BÖTTCHER, Arnd, D-67227 Frankenthal (DE); HENKELMANN, Jochem, D-68165 Mannheim (DE); PREISS, Thomas, D-67065 Ludwigshafen (DE); BRUNNER, Melanie, D-67105 Schifferstadt (DE)
(74) Vertreter: Kinzebach, Werner, Dr.
(86) Internationale Anmeldenummer: EP9807785
(87) Internationale Veröffentlichungsnummer: WO9928034

(56) Entgegenhaltungen:
- EP-A- 0 827 949
- DE-A- 2 719 745
- DE-B- 1 191 364
- US-A- 3 462 507
- US-A- 4 584 418

## Beschreibung

Die vorliegende Erfindung betrifft ein Verfahren zur Herstellung eines Katalysators durch Aktivierung einer katalytischen Masse, welche wenigstens eine Verbindung eines Metalls der ersten oder zweiten Nebengruppe des Periodensystems, gegebenenfalls Bismut oder eine Verbindung davon, und gegebenenfalls einen Träger umfasst sowie die nach diesem Verfahren erhältlichen Katalysatoren. Die Erfindung betrifft weiterhin ein Verfahren zur Herstellung von Alkinolen und ein Verfahren zur Herstellung von Aminoalkinen unter Verwendung dieser Katalysatoren.

Die Herstellung von Alkinolen durch Addition einer Carbonylverbindung an ein Alkin und insbesondere an Acetylen unter Erhalt der Dreifachbindung sowie die Herstellung von Aminoalkinen durch Umsetzung eines Alkins mit einer Carbonylverbindung und einem Amin im Sinne einer Mannich-Kondensation sind seit langem bekannt und werden industriell genutzt. Beide Reaktionen können homogen oder heterogen katalysiert werden, wobei im Allgemeinen Schwermetallacetylide und insbesondere Katalysatoren auf Basis von Kupfer(I)acetylid eingesetzt werden.

Die US-A-3,496,232 beschreibt die Herstellung von N-Alkyl-substituierten Aminoalkinen in einer homogen oder heterogen katalysierten Mannich-Reaktion, wobei als Katalysatoren Salze von Metallen der ersten oder zweiten Nebengruppe, wie z. B. die Chloride, Acetate, Formiate und Acetylide und speziell Kupferacetylid eingesetzt werden. Diese können im Falle der heterogen katalysierten Verfahrensvariante auf einen inerten Träger eingesetzt werden. Die Herstellung des Kupferacetylid-Katalysators erfolgt dabei vor der eigentlichen Mannich-Reaktion durch Umsetzung von Kupfer(II)chlorid mit Paraformaldehyd und Acetylen in einem Autoklaven.

Die DE-A-23 14 693 beschreibt ein Verfahren zur Herstellung von Butindiol durch Umsetzung von Acetylen und Formaldehyd, wobei ein geträgerter Schwermetallacetylidkatalysator eingesetzt wird, der durch Tränken eines Trägers mit einer Schwermetallsalzlösung und anschließendes Behandeln mit gasförmigem Acetylen erhalten wird.

Die DE-A-24 21 407 beschreibt ebenfalls ein Verfahren zur Herstellung von Butindiol, wobei geträgerte Kupferacetylidkatalysatoren eingesetzt werden.

Die DE-A-26 02 418 beschreibt ein Verfahren zur Herstellung von Butindiol, wobei ein geträgerter Katalysator eingesetzt wird, der durch Reaktion einer Kupfer(II)-Verbindung mit Acetylen und Formaldehyd in wässriger Lösung bei einem pH-Wert unterhalb von 5,5 erhalten wird.

Die US-A-3,650,985 beschreibt die Herstellung trägerloser Kupferacetylid-Katalysatoren, der allgemeinen Formel (CuC₂)_{w} (CH₂O)ₓ (C₂H₂)_{y} (H₂O)_{z} mit 1 ≤ w, x, y < 100, vorzugsweise w = 4, x = 0,24 bis 4, y = 0,24 bis 4 und z = 0,67 bis 2,8. Diese Katalysatoren können zusätzlich eine Bismutverbindung enthalten und sind durch simultanes Einwirken von Formaldehyd und Acetylen auf eine partikuläre, wasserunlösliche Kupferverbindung, vorzugsweise basisches Kupfercarbonat, wie z.B. synthetisch hergestellter Malachit herstellbar. Sie werden als wässrige Katalysatoraufschlämmung zur Ethinylierung acetylenisch ungesättigter Kohlenwasserstoffe verwendet. Ähnliche Malachit-Katalysatoren werden in der US-A-3,560,576 beschrieben.

Die US-A-4,110,249 beschreibt ein Verfahren zur Herstellung von Bismut-modifizierten, kugelförmigen Malachiten und ihre Umsetzung mit Acetylen und Formaldehyd zu trägerlosen Ethinylierungskatalysatoren. Die US-A-4,127,734 beschreibt ein Verfahren zur Herstellung von 1,4-Butindiol durch Umsetzung von Acetylen und Formaldehyd in Gegenwart dieser Katalysatoren.

Die US-A-4,536,491 beschreibt Agglomerate kugelförmiger Malachite, die Bismut und Kieselsäure umfassen. Diese Malachite können durch Umsetzung in Form einer wässrigen Aufschlämmung mit Acetylen und Formaldehyd in Kupferacetylid-Komplexe überführt werden. Die US-A-4,584,418 beschreibt ein Verfahren zur Herstellung von 1,4-Butindiol durch Umsetzung von Acetylen mit Formaldehyd in Gegenwart dieser Katalysatoren.

Die US-A-4,119,790 beschreibt ein kontinuierliches, mehrstufiges Niederdruck-Ethinylierungsverfahren zur Herstellung von 1,4-Butindiol, wobei ein Ethinylierungskatalysator auf der Basis eines wasserunlöslichen Kupferacetylid-Komplexes eingesetzt wird. Die Herstellung dieses Ethinylierungskatalysators erfolgt durch Umsetzung eines Precursors, welcher zwischen 20 und 35 Gew.-% Kupfer und 0 bis 3 Gew.-% Bismut auf einem Magnesiumsilicatträger umfasst, mit Formaldehyd in Gegenwart von Acetylen.

Die EP-A-0 080 794 beschreibt ein heterogen katalysiertes Verfahren zur Herstellung von N,N-disubstituierten Propinylaminen, wobei als Katalysatoren Kupferacetylide auf einem mit Bismutoxid dotierten Magnesiumsilicatträger eingesetzt werden. Dabei handelt es sich um übliche Ethinylierungskatalysatoren, welche vor ihrem Einsatz mit Acetylen und Formaldehyd aktiviert werden müssen.

Nachteilig an den zuvor beschriebenen Katalysatoren ist, dass zu ihrer Herstellung eine katalytische Masse unter Einsatz von Acetylen aktiviert wird. Das Arbeiten mit Acetylen, einem thermisch instabilen, bereits bei Normaldruck leicht explodierenden Gas, ist technisch aufwendig, da erhebliche sicherheitstechnische Maßnahmen zur Auslegung der Reaktoren erforderlich sind. Dies gilt insbesondere für das Arbeiten mit verflüssigtem Acetylen und/oder dessen Handhabung bei hohen Drücken. Auch die bei der Aktivierung der katalytischen Massen mit Acetylen erhaltenen Schwermetallkatalysatoren neigen im Allgemeinen zur explosionsartigen Zersetzung und sind daher nur schwer zu handhaben. Verfahren, bei denen derartige Katalysatoren eingesetzt werden, sind daher wirtschaftlich benachteiligt. Dies gilt insbesondere dann, wenn nur bei der Herstellung bzw. Aktivierung des Katalysators Acetylen eingesetzt wird, während die nachfolgende Ethinylierungs- oder Aminoalkylierungsreaktion unter Einsatz eines anderen Alkins erfolgt.

Der vorliegenden Erfindung liegt daher die Aufgabe zugrunde, ein Verfahren zur Herstellung eines Katalysators durch Aktivierung einer katalytischen Masse zur Verfügung zu stellen, wobei auf den Einsatz von Acetylen verzichtet werden kann.

Überraschenderweise wurde nun gefunden, dass diese Aufgabe durch ein Verfahren gelöst wird, bei dem eine katalytische Masse, welche wenigstens ein Metall der ersten oder zweiten Nebengruppe oder eine Verbindung davon, gegebenenfalls Bismut oder eine Verbindung davon, und gegebenenfalls einen Träger umfasst, mit einem von Acetylen verschiedenen Alkin und einer Carbonylverbindung aktiviert wird.

Überraschenderweise wurde weiterhin gefunden, dass auf den Einsatz von Acetylen bei der Aktivierung der katalytischen Masse im Allgemeinen auch dann verzichtet werden kann, wenn die folgende Alkinylierungs- oder Aminoalkylierungsreaktion in Gegenwart von Acetylen durchgeführt wird, da das zur Aktivierung eingesetzte Alkin im Allgemeinen ein anderes Alkin als das zur Folgereaktion eingesetzte sein kann.

Gegenstand der Erfindung ist ein Verfahren zur Herstellung eines Katalysators durch Aktivierung einer katalytischen Masse, welche
a) wenigstens ein Metall der ersten oder zweiten Nebengruppe oder eine Verbindung davon,
b) gegebenenfalls einen Träger
umfasst, das dadurch gekennzeichnet ist, dass man die Masse mit einem Alkin der allgemeinen Formel I

R¹-C≡C-R² (I)

worin
- R¹: für Alkyl, Cycloalkyl, Aryl, Hydroxyalkyl, Halogenalkyl, Alkoxy oder Alkoxyalkyl,
- R²: für ein Wasserstoffatom, Alkyl, Cycloalkyl oder Aryl steht,
und einer Carbonylverbindung der allgemeinen Formel II 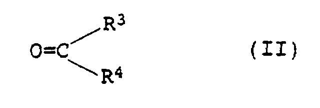 worin
- R³ und R⁴: unabhängig voneinander für ein Wasserstoffatom, Alkyl, Halogenalkyl, Cycloalkyl oder Aryl stehen,
behandelt.

Im Rahmen der vorliegenden Erfindung steht Halogen für Fluor, Chlor, Brom und Jod und insbesondere für Chlor und Brom.

Der Ausdruck "Alkyl" umfasst geradkettige und verzweigte Alkylgruppen. Vorzugsweise handelt es sich dabei um geradkettige oder verzweigte C₁-C₁₂-Alkyl- und insbesondere C₁-C₆-Alkylgruppen. Beispiele für Alkylgruppen sind insbesondere Methyl, Ethyl, Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 1-Ethylpentyl, 2-Ethylpentyl, 1-Propylbutyl, Octyl, Decyl und Dodecyl.

Halogenalkyl steht für eine wie oben definierte Alkylgruppe, die mit einem oder mehreren Halogenatomen, insbesondere Chlor und Brom, teilweise oder vollständig, vorzugsweise mit ein bis drei Halogenatomen, halogeniert ist.

Die obigen Ausführungen zur Alkylgruppe und Halogenalkylgruppe gelten in entsprechender Weise für die Alkylgruppe in Alkoxy-, Alkoxyalkyl- und Hydroxyalkyl-Resten.

Cycloalkyl steht vorzugsweise für C₃-C₈-Cycloalkyl, wie Cyclopropyl, Cyclobutyl, Cyclopentyl, Cyclohexyl, Cycloheptyl oder Cyclooctyl, oder Cyclopentylmethyl, Cyclopentylethyl und Cyclohexylmethyl und Cyclohexylethyl.

Aryl steht vorzugsweise für Phenyl oder Naphthyl.

Vorzugsweise verwendet man im erfindungsgemäßen Verfahren eine katalytische Masse, welche als Komponente a) Kupfer oder eine Kupferverbindung umfasst. Geeignete Kupferverbindungen a) sind z. B. ausgewählt unter Kupferhalogeniden, wie Kupfer(I)chlorid, Rupfer(II)chlorid, Kupfer(I)bromid und Kupfer(II)bromid, Kupfer(I)sulfat, Kupfer(II)sulfat, Kupfer(II)hydroxid, Kupfer(I)oxid, Kupfer(II)oxid, Kupferphosphaten, Kupfersilicaten, basischen Kupfercarbonaten etc., und Mischungen davon. Bevorzugte Kupferverbindungen a) sind Kupfer(II)oxid und basische Kupfercarbonate, wie natürliche und insbesondere bevorzugt synthetische Malachite.

Geeignete Bismutverbindungen sind z. B. ausgewählt unter Bismutoxid Bi₂O₃, Bismutoxidcarbonat (BiO)₂CO₃, Bismutnitrat Bi(NO₃)₃ und Bismutoxidnitrat BiO(NO₃). Bevorzugt wird als Komponente b) Bismutoxid eingesetzt.

Die im erfindungsgemäßen Verfahren zur Aktivierung eingesetzten katalytischen Massen können gegebenenfalls einen Träger c) umfassen, der insbesondere ausgewählt ist unter Aluminiumoxid, Alumosilicaten, Zirkoniumoxid, Titandioxid und vorzugsweise Siliciumdioxid.

Vorzugsweise wird zur Aktivierung eine katalytische Masse eingesetzt, welche 10 bis 20 Gew.-% Kupfer(II)oxid, 1 bis 5 Gew.-% Bismutoxid und 75 bis 89 Gew.-% Siliciumdioxid als Träger umfasst.

Zur Aktivierung geeignete, geträgerte katalytische Massen sind aus dem Stand der Technik bekannt und werden z. B. in der US-A-3,650,985, der DE-A-26 02 418 und in der US-A-4,119,790 beschrieben. Auf die Offenbarung dieser Druckschriften wird hiermit Bezug genommen, wobei die Aktivierung der dort beschriebenen, geträgerten katalytischen Massen im erfindungsgemäßen Verfahren nicht unter Einsatz von Acetylen erfolgt.

Gemäß einer bevorzugten Ausführungsform der vorliegenden Erfindung wird zur Aktivierung eine trägerlose katalytische Masse eingesetzt, welche ein Bi-dotiertes Malachit umfasst. Derartige katalytische Massen sind z. B. in der US-A-4,110,249, der DE-A-25 08 084 und in der US-A-4,536,491 beschrieben. Auf die Offenbarung dieser Druckschriften wird hiermit ebenfalls Bezug genommen, wobei die Aktivierung der dort beschriebenen, trägerlosen katalytischen Massen im erfindungsgemäßen verfahren wiederum nicht unter Einsatz von Acetylen erfolgt.

Zur Aktivierung werden die zuvor beschriebenen, geträgerten oder trägerlosen katalytischen Massen mit einem Alkin der allgemeinen Formel I und einer Carbonylverbindung der allgemeinen Formel II behandelt. Die Temperatur liegt dabei im Allgemeinen in einem Bereich von etwa 0 bis 150 °C, bevorzugt etwa 20 bis 100 °C. Der pH-Wert des zur Behandlung der katalytischen Massen eingesetzten Reaktionsmediums liegt im Allgemeinen im sauren oder neutralen Bereich, wie z. B. bei pH-Werten von etwa 4,0 bis 7,0, vorzugsweise im leicht sauren Bereich, wie z. B. bei pH-Werten von etwa 5,5 bis 6,9. Zur Einstellung des pH-Wertes können übliche Basen, wie Alkalihydroxide, z. B. NaOH und KOH, Alkalicarbonate, z. B. Na₂CO₃ und K₂CO₃, und Alkalihydrogencarbonate, z. B. NaHCO₃ und KHCO₃, eingesetzt werden. Vorzugsweise wird die katalytische Masse als Aufschlämmung in Wasser aktiviert. Es können jedoch auch Mischungen aus Wasser und mindestens einem vollständig wassermischbaren, gegenüber den Reaktanden inerten organischen Lösungsmittel eingesetzt werden. Geeignete Lösungsmittel sind z. B. gesättigte cyclische Ether, wie Tetrahydrofuran und Dioxan.

Der Druck bei der Aktivierung der katalytischen Massen ist im Allgemeinen geringer als bei den aus dem Stand der Technik bekannten Verfahren, bei denen Acetylen zur Aktivierung eingesetzt wird. Im Allgemeinen beträgt der Druck bei dem erfindungsgemäßen Verfahren bis zu etwa 3 bar, bevorzugt bis zu etwa 2 bar. Nach einer insbesondere bevorzugten Ausführungsform erfolgt die Aktivierung der katalytischen Massen bei Umgebungsdruck.

Wenn als Alkin der allgemeinen Formel I ein unter den Reaktionsbedingungen gasförmiges Alkin, wie z. B. Propin oder Butin, eingesetzt wird, so kann die Aktivierung gewünschtenfalls unter Eigendruck bei Einhaltung der vorbeschriebenen Druckbedingungen durchgeführt werden. Nach einer möglichen Reaktionsweise kann dann die Carbonylverbindung der allgemeinen Formel II gemeinsam mit der trägerlosen oder geträgerten katalytischen Masse und gegebenenfalls mit einem Lösungsmittel in einem mit einer Mischvorrichtung versehenen Reaktor vorgelegt werden. Geeignete Reaktoren sind dem Fachmann bekannt. Zu ihnen zählen die in Ullmanns Enzyklopädie der technischen Chemie, 3. Auflage, Band 1, S. 117 f. und S. 769 f (1951) beschriebenen Reaktionsbehälter für Reaktionen unter Druck. Die Zugabe des gasförmigen Alkins erfolgt vorzugsweise unterhalb des Flüssigkeitsspiegels der vorgelegten Aktivierungsmischung, z. B. mit einem Tauchrohr oder mit einer Rohrschlange, die Öffnungsbohrungen in oder gegen die Strömungsrichtung der Reaktionsmischung aufweist. Die Zugabegeschwindigkeit wird durch die vorgenannten einzuhaltenden Druckbereiche limitiert.

wird als Alkin der allgemeinen Formel I ein unter den Reaktionsbedingungen flüssiges oder festes Alkin eingesetzt, so kann die Aktivierung im Allgemeinen bei Umgebungsdruck in einem dafür üblichen Reaktor mit einer Mischvorrichtung, z. B. einem Rührreaktor, erfolgen. Die Aktivierung kann dann z. B. in Batch-Fahrweise erfolgen, wobei vorzugsweise die Carbonylverbindung mit einer Aufschlämmung der trägerlosen oder geträgerten katalytischen Masse in Wasser und gegebenenfalls einem Lösungsmittel im Reaktor vorgelegt und das Alkin in Substanz oder als Lösung nach Maßgabe seines Verbrauchs dem Reaktor zugeführt wird.

Gemäß einer bevorzugten Ausführungsform des erfindungsgemäßen Verfahrens werden zur Aktivierung der katalytischen Massen Alkine der Formel I eingesetzt, worin der Rest R¹ für Alkyl oder Hydroxyalkyl steht. Vorzugsweise steht der Rest R² für ein Wasserstoffatom oder für Alkyl. Dazu zählen z. B. Propin, 1-Butin, 1-Pentin, 1-Hexin, 1-Heptin, 1-Octin, Propinol, 3-Butin-1-ol, 3-Butin-2-ol, 2-Methyl-3-butin-2-ol, 4-Pentin-1-ol, 4-Pentin-2-ol, 4-Pentin-3-ol, 2-Methyl-4-pentin-2-ol, 3-Methyl-4-pentin-2-ol, 3-Methyl-4-pentin-3-ol etc. Bevorzugt werden 3-Butin-2-ol, 2-Methyl-3-butin-2-ol und 1-Hexin eingesetzt.

Gemäß einer bevorzugten Ausführungsform des erfindungsgemäßen Verfahrens werden zur Aktivierung der katalytischen Massen Carbonylverbindungen der allgemeinen Formel II eingesetzt, worin R³ und R⁴ unabhängig voneinander für ein Wasserstoffatom oder Alkyl stehen. Dazu zählen z. B. Acetaldehyd, Propionaldehyd, n-Butyraldehyd, n-Valeraldehyd etc. Insbesondere bevorzugt wird Formaldehyd oder ein Kondensationsprodukt davon, z. B. Paraformaldehyd, eingesetzt.

Das erfindungsgemäße Verfahren eignet sich nicht nur zur Herstellung eines Katalysators durch Aktivierung einer katalytischen Masse, sondern vorteilhafterweise auch zur Reaktivierung eines zuvor verwendeten, deaktivierten Katalysators.

Ein weiterer Gegenstand der Erfindung sind die Katalysatoren, die durch das zuvor beschriebene Verfahren erhältlich sind. Bei der Herstellung dieser Katalysatoren nach dem erfindungsgemäßen Verfahren kann auf eine Aktivierung der katalytischen Massen mit Acetylen verzichtet werden. Dies ist insbesondere dann von Vorteil, wenn der resultierende Katalysator in einem Verfahren eingesetzt werden soll, in dem ebenfalls kein Acetylen als Reaktand verwendet wird. Somit können derartige Verfahren mit den erfindungsgemäßen Katalysatoren im Allgemeinen wirtschaftlicher gestaltet werden, da die für das Arbeiten mit Acetylen erforderlichen Sicherheitsmaßnahmen eingespart werden können. Dies gilt insbesondere dann, wenn zur Aktivierung der katalytischen Massen keine gasförmigen Alkine eingesetzt werden, so dass diese drucklos durchgeführt werden kann. Im Gegensatz zu den bisher als Katalysatoren eingesetzten Kupferacetyliden neigen die erfindungsgemäßen Katalysatoren im Allgemeinen auch nicht zur explosiven Zersetzung und lassen sich somit gefahrlos handhaben. Die Aktivität dieser Katalysatoren ist im Allgemeinen zumindest vergleichbar mit derjenigen der mit Acetylen aktivierten Katalysatoren. Insbesondere bei der Alkinylierung von Alkinen bzw. Hydroxyalkinen mit terminalen Dreifachbindungen weisen die erfindungsgemäßen Katalysatoren allgemein eine gleich gute oder bessere Aktivität als entsprechende, mit Acetylen aktivierte Katalysatoren auf. Dies gilt speziell auch bei Ethinylierungsreaktionen unter Verwendung von Acetylen. Auch wenn, wie in diesem Falle, für die katalysierte Reaktion Acetylen eingesetzt wird, ist es von Vorteil, zur Aktivierung des Katalysators ein von Acetylen verschiedenes Alkin einzusetzen. Wie zuvor beschrieben, neigen die erfindungsgemäßen Katalysatoren im Allgemeinen nicht zur explosionsartigen zersetzunq und lassen sich somit leichter handhaben und lagern. Somit entfallen zumindest für die Aktivierung der katalytischen Massen die für das Arbeiten mit Acetylen erforderlichen Sicherheitsvorkehrungen.

Ein weiterer Gegenstand der Erfindung ist ein Verfahren zur Herstellung von Alkinolen der allgemeinen Formel III 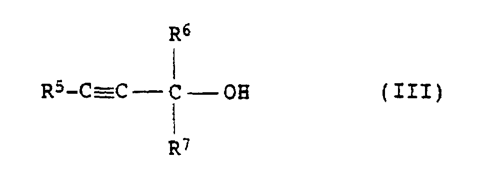 worin
- R⁵: für ein Wasserstoffatom, Alkyl, Halogenalkyl, Cycloalkyl, Aryl, Alkoxy, Alkoxyalkyl oder einen -C(R⁶R⁷)OH-Substituenten steht,
- R⁶ und R⁷: unabhängig voneinander für ein Wasserstoffatom, Alkyl, Halogenalkyl, Cycloalkyl oder Aryl stehen,
wobei man ein Gemisch aus einem Alkin der allgemeinen Formel IV

R⁵-C≡C-H (IV)

worin
- R⁵: die oben angegebenen Bedeutungen besitzt, und
einer Carbonylverbindung der allgemeinen Formel V 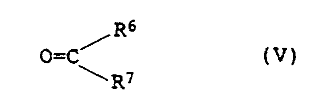 worin
- R⁶ und R⁷: die oben angegebenen Bedeutungen besitzen,
umsetzt, das dadurch gekennzeichnet ist, dass die Umsetzung in Gegenwart eines erfindungsgemäßen Katalysators erfolgt.

Gemäß einer bevorzugten Ausführungsform des erfindungsgemäßen Verfahrens werden Verbindungen hergestellt, worin der Rest R⁵ für ein Wasserstoffatom, Alkyl oder einen -C(R⁶R⁷)OH-Substituenten steht. vorzugsweise stehen die Reste R⁶ und R⁷ unabhängig voneinander für ein Wasserstoffatom oder Alkyl. Insbesondere stehen die Reste R⁶ und R⁷ beide für Wasserstoff.

Zur Herstellung von Verbindungen der Formel III, worin R⁵ für Wasserstoff steht, wird als Alkin der Formel IV Acetylen eingesetzt. Das Molmengenverhältnis von Acetylen zu Carbonylverbindung der Formel V beträgt dann etwa 1:0,5 bis etwa 1:1, so dass im Wesentlichen Monoadditionsprodukte resultieren.

Zur Herstellung von Verbindungen der Formel III, worin R⁵ für einen -C(R⁶R⁷)OH-Substituenten steht, wird als Alkin der Formel IV ebenfalls Acetylen eingesetzt. Das Molmengenverhältnis von Acetylen zu Carbonylverbindung der Formel V beträgt dann etwa 1:2 bis 1:4, so dass im Wesentlichen Bisadditionsprodukte resultieren.

Vorzugsweise handelt es sich bei dem Alkinol der Formel III um Propinol, But-2-in-1-ol, But-2-in-1,4-diol, But-3-in-2-ol, Hex-3-in-2,5-diol, Pent-3-in-2-ol etc.

Ein weiterer Gegenstand der Erfindung ist ein Verfahren zur Herstellung von Aminoalkinen der allgemeinen Formel VI 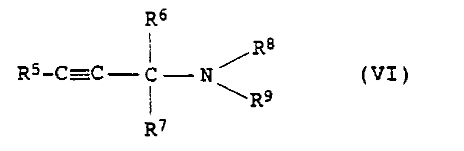 worin
- R⁵, R⁶ und R⁷: die oben angegebenen Bedeutungen besitzen,
- R⁸ und R⁹: unabhängig voneinander für ein Wasserstoffatom, Alkyl, Halogenalkyl, Cycloalkyl, Aryl oder Hydroxyalkyl stehen, oder
- R⁸ und R⁹: zusammen mit dem Stickstoffatom, an das sie gebunden sind, einen 5- oder 6-gliedrigen, heterocyclischen Ring bilden;
wobei man ein Gemisch aus einem Alkin der allgemeinen Formel IV

R⁵-C≡C-H (IV)

worin
- R⁵: die oben angegebenen Bedeutungen besitzt,
einer Carbonylverbindung der allgemeinen Formel V 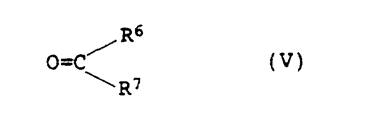 worin
- R⁶ und R⁷: die oben angegebenen Bedeutungen besitzen, und
einem Amin der allgemeinen Formel VII 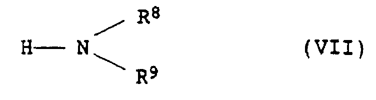 worin
- R⁸ und R⁹: die oben angegebenen Bedeutungen besitzen,
umsetzt, das dadurch gekennzeichnet ist, dass die Umsetzung in Gegenwart eines erfindungsgemäßen Katalysators erfolgt.

Die Reste R⁸ und R⁹ können zusammen mit dem Stickstoffatom, an das sie gebunden sind, einen heterocyclischen Ring bilden. Beispiele hierfür sind Succinimido-, Phthalimido-Gruppen oder ein ungesättigter oder gesättigter, 5- oder 6-gliedriger, heterocyclischer Ring, der gegebenenfalls ein weiteres Heteroatom, ausgewählt unter S und N, vorzugsweise N, enthält. Als Beispiele können hierfür genannt werden: Piperidinyl-, Piperazinyl- und Tetrahydropyrimidinyl-Gruppen.

Gemäß einer bevorzugten Ausführungsform des erfindungsgemäßen Verfahrens werden Verbindungen hergestellt, worin der Rest R⁵ für ein Wasserstoffatom, Alkyl oder einen -C(R⁶R⁷)OH-Substituenten steht. Vorzugsweise stehen R⁶ und R⁷ unabhängig voneinander für ein Wasserstoffatom oder Alkyl. Insbesondere stehen die Reste R⁶ und R⁷ beide für Wasserstoff. Weiterhin bevorzugt stehen die Reste R⁸ und R⁹ unabhängig voneinander für ein Wasserstoffatom, Alkyl oder Hydroxyalkyl. Geeignete Amine der Formel VII sind dann z. B. Dimethylamin, Diethylamin, Di-n-propylamin, Diisopropylamin, Di-n-butylamin, Diethanolamin, Dipropanolamin etc.

Zur Herstellung von Verbindungen der Formel VI, worin R⁵ für Wasserstoff steht, wird als Alkin der Formel IV Acetylen eingesetzt. Das Molmengenverhältnis von Acetylen zu Carbonylverbindung der Formel V und Amin der Formel VII beträgt dann etwa 1:0,5:0,5 bis etwa 1:1:1, so dass im Wesentlichen Monoadditionsprodukte resultieren.

Zur Herstellung von Verbindungen der Formel VI, worin R⁵ für einen -C(R⁶R⁷)OH-Substituenten steht, wird als Alkin der Formel IV ebenfalls Acetylen eingesetzt. Das Molmengenverhältnis von Acetylen zu Carbonylverbindung der Formel V und Amin der Formel VII beträgt dann etwa 1:2:2 bis etwa 1:4:4, so dass im Wesentlichen Bisadditionsprodukte resultieren.

Vorzugsweise handelt es sich bei dem Aminoalkin der Formel VI um N,N-Dimethylaminopropin, N,N-Diethylaminopropin, N,N-Dihydroxyethylaminopropin, N,N-Dimethylaminobut-2-in, N,N-Diethylaminobut-2-in, N,N-Dihydroxyethylaminobut-2-in, 5-(N,N-Dimethylamino)-pent-3-in-2-ol und insbesondere um 5-(N,N-Diethylamino)-pent-3-in-2-ol.

Die zur Herstellung der erfindungsgemäßen Katalysatoren eingesetzten Alkine der Formel I können nach einer Ausführungsform den in der katalysierten Alkinylierungs- bzw. Aminoalkylierungsreaktion eingesetzten Alkinen der allgemeinen Formel IV entsprechen. In diesem Falle können die Katalysatoren im Allgemeinen unmittelbar vor der Alkinylierung bzw. der Aminoalkylierung hergestellt werden, wobei auf eine Isolierung und gegebenenfalls eine Reinigung des Katalysators verzichtet werden kann. Vorzugsweise erfolgt die Herstellung des Katalysators und die mit ihm durchgeführte Folgereaktion in einem Reaktor in einer Eintopfreaktion.

Nach einer weiteren Ausführungsform kann zur Herstellung der erfindungsgemäßen Katalysatoren jedoch auch ein Alkin der Formel I eingesetzt werden, welches von den in der katalysierten Alkinylierungs- bzw. Aminoalkylierungsreaktion eingesetzten Alkinen der Formel IV verschieden ist. Dies ist, wie zuvor beschrieben, insbesondere dann von Bedeutung, wenn zur Alkinylierung bzw. zur Aminoalkylierung Acetylen als Alkin eingesetzt wird. In diesem Falle kann zur Herstellung des Katalysators ein von Acetylen verschiedenes Alkin eingesetzt werden und die Herstellung des Katalysators kann gewünschtenfalls in einem separaten Reaktor durchgeführt werden. Der resultierende Katalysator kann nach üblichen Verfahren isoliert und gereinigt werden, z. B. durch Waschen mit Wasser.

Ein weiterer Gegenstand der Erfindung ist die Verwendung der erfindungsgemäßen Katalysatoren bei Alkinylierungsreaktionen und bei der Herstellung von Aminoalkinen durch Umsetzung eines Alkins mit einer Carbonylverbindung und einem Amin.

Die Erfindung wird anhand der folgenden, nicht einschränkenden Beispiele näher erläutert.

### Beispiele

1) Aktivierung einer trägerlosen katalytischen Masse auf Basis von Kupfermalachit:
   Die Herstellung einer katalytischen Masse mit einem Bi-Gehalt von 4 % erfolgt wie in der DE-A-25 08 084 beschrieben.
   In einem 500 ml-Glasreaktor mit Stromstörern und Scheibenrührer, Rückflusskühler, Temperaturmessung, pH-Elektrode und Zufuhr von wässriger NaHCO₃-Lösung über ein Tauchrohr werden unter Stickstoff 377,5 g Formaldehyd (49%ig in Wasser) vorgelegt, auf 70 °C erhitzt und mit NaHCO₃-Lösung auf einen pH-Wert von 6,5 eingestellt (die Temperatur sinkt dabei leicht). Man erwärmt weiter, bis im Reaktor eine Temperatur von 75 °C erreicht ist, dann werden 9,7 g Kupfermalachit-Katalysator in 50 g Wasser aufgeschlämmt und in den Reaktor überführt. Über einen Zeitraum von 4 h werden nun kontinuierlich unter Rühren 70,1 g 3-Butin-2-ol (55%ig in Wasser) mittels einer HPLC-Pumpe zudosiert, hierbei färbt sich der Katalysator im Verlauf einiger Stunden dunkel. Fällt der pH-Wert im Verlauf der Aktivierungsreaktion unter einen Wert von 6, wird zum Ausgleich 2%ige, wässrige NaHCO₃-Lösung zugegeben (insgesamt 372 ml). Zum Beenden der Aktivierung wird nach 8 h langsam auf 25 °C abgekühlt. Anschließend wird der Katalysator abzentrifugiert und zweimal mit Wasser gewaschen.
2) Aktivierung einer geträgerten katalytischen Masse auf Basis von Kupferoxid
   Die Herstellung der katalytischen Masse in Form von Strängen erfolgt wie in der DE-A-26 02 418 beschrieben.
   In einem 500 ml-Glasreaktor mit Stromstörern und Scheibenrührer, Rückflusskühler, Temperaturmessung, pH-Elektrode und Zufuhr von wässriger NaHCO₃-Lösung über ein Tauchrohr werden unter Stickstoff 377,5 g Formaldehyd (49%ig in Wasser) vorgelegt, auf 70 °C erhitzt und mit NaHCO₃-Lösung auf einen pH-Wert von 6,5 eingestellt (die Temperatur sinkt dabei leicht). Man erwärmt weiter, bis im Reaktor eine Temperatur von 75 °C erreicht ist (nach ca. 15 min), dann werden 30 g geträgerter Kupfer-Katalysator (Stränge, ca. 15 % CuO, 80 % SiO₂ und 4 % Bi₂O₃, Durchmesser etwa 3 mm, Länge etwa 6 bis 8 mm) in 50 g Wasser aufgeschlämmt und in den Reaktor überführt. Über einen Zeitraum von 2,5 h werden nun kontinuierlich unter Rühren 70,1 g 3-Butin-2-ol (55%ig in Wasser) mittels einer HPLC-Pumpe zudosiert. Fällt der pH-Wert im Verlauf der Aktivierungsreaktion unter 6, wird zum Ausgleich 2%ige, wässrige NaHCO₃-Lösung zugegeben (insgesamt 100 ml). Zum Beenden der Aktivierung wird nach 20 h langsam auf 25 °C abgekühlt. Anschließend wird der Katalysator abfiltriert und zweimal mit Wasser gewaschen.
3) Aktivierung einer trägerlosen katalytischen Masse auf Basis von Kupfermalachit:
   Es wird analog zu der Vorschrift aus Beispiel 1) verfahren, wobei anstelle des 3-Butin-2-ols 56,5 g Propargylalkohol (55%ig in Wasser) mittels einer HPLC-Pumpe zudosiert werden. Zur Einstellung des pH-Wertes werden insgesamt 350 ml NaHCO₃-Lösung zugegeben.
4) Synthese von Diethylaminopentinol unter Einsatz des Katalysators aus Beispiel 1
   In einer 250 ml-Rührapparatur werden 70,1 g (0,55 mol) 3-Butin-2-ol (55%ige Lösung in Wasser), 30,3 g Paraformaldehyd (1 mol) und 10 g des aktivierten Malachitkatalysators aus Beispiel 1 vorgelegt. Nach Erwärmen auf 80 °C werden unter Rühren 73,1 g (1 mol) Diethylamin innerhalb 1 h zugetropft. Das Reaktionsgemisch wird noch 22,5 h bei 80 °C gerührt, danach wird langsam abgekühlt. Der Austrag wird zentrifugiert, vom Katalysator befreit und destillativ aufgearbeitet (Ausbeute: 85 %). Der Katalysator kann nach Spülen mit Wasser erneut eingesetzt werden.
5) Synthese von Diethylaminobutinol unter Einsatz des Katalysators aus Beispiel 3
   In einer 250 ml-Rührapparatur werden 56,1 g (0,55 mol) Propargylalkohol (55%ige Lösung in Wasser), 30,3 g Paraformaldehyd (1 mol) und 5,1 g des aktivierten Malachitkatalysators aus Beispiel 3 vorgelegt. Nach Erwärmen auf 80 °C werden unter Rühren 73,1 g (1 mol) Diethylamin innerhalb 1 Stunde zugetropft. Das Reaktionsgemisch wird noch 22,5 Stunden bei 80 °C gerührt, danach wird langsam abgekühlt. Der Austrag wird zentrifugiert, vom Katalysator befreit und destillativ aufgearbeitet (Ausbeute 92 %). Der Katalysator kann nach Spülen mit Wasser erneut eingesetzt werden.
6) Synthese von Butindiol unter Einsatz des Katalysators aus Beispiel 3
   In einer 250 ml-Rührapparatur werden 56,1 g (0,55 mol) Propargylalkohol (55%ige Lösung in Wasser), 101 g (1 mol) Formaldehyd (30%ige Lösung in Wasser) und 5,1 g des aktivierten Malachit-Katalysators aus Beispiel 3 vorgelegt. Nach Erwärmen auf 90 °C wird das Reaktionsgemisch noch 22,5 Stunden bei 90 °C gerührt, danach wird langsam abgekühlt. Der Austrag wird zentrifugiert, vom Katalysator befreit und gaschromatographisch (30 m DB1-Säule, Injektortemperatur 260 °C, Detektortemperatur 280 °C, Gerät Hewlett Packard HP 580 90A, Temperaturprogramm: Anfangstemperatur 80 °C, Aufheizgeschwindigkeit 10 °C/min, Endtemperatur 280 °C) analysiert (Ausbeute 80 %).

## Patentansprüche

1. Verfahren zur Herstellung eines Katalysators durch Aktivierung einer katalytischen Masse, welche
a) wenigstens ein Metall der ersten oder zweiten Nebengruppe oder eine Verbindung davon,
b) gegebenenfalls einen Träger
umfasst, **dadurch gekennzeichnet, dass** man die Masse mit einem Alkin der allgemeinen Formel I
R¹-C≡C-R² (I)
worin
R¹ für Alkyl, Cycloalkyl, Aryl, Hydroxyalkyl, Halogenalkyl, Alkoxy oder Alkoxyalkyl,
R² für ein Wasserstoffatom, Alkyl, Cycloalkyl oder Aryl steht,
und einer Carbonylverbindung der allgemeinen Formel II 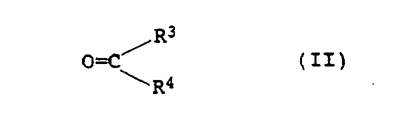 worin
R³ und R⁴ unabhängig voneinander für ein Wasserstoffatom, Alkyl, Halogenalkyl, Cycloalkyl oder Aryl stehen,
behandelt.

2. Verfahren nach Anspruch 1, **dadurch gekennzeichnet, dass** die Behandlung der katalytischen Masse bei einem Druck von bis zu 3 bar, bevorzugt bis zu 2 bar, insbesondere bevorzugt bei Umgebungsdruck, erfolgt.

3. Verfahren nach einem der vorhergehenden Ansprüche, **dadurch gekennzeichnet, dass** man als Komponente a) Kupfer oder eine Kupferverbindung, bevorzugt ausgewählt unter Kupferoxid und Malachit, einsetzt.

4. Verfahren nach einem der vorhergehenden Ansprüche, **dadurch gekennzeichnet, dass** die katalytische Masse auch Bismut oder eine Bismutverbindung enthält, die vorzugsweise ausgewählt ist unter Bi₂O₃, (BiO)₂CO₃, Bi(NO₃)₃ und BiO(NO₃).

5. Verfahren nach einem der vorhergehenden Ansprüche, **dadurch gekennzeichnet, dass** man eine katalytische Masse einsetzt, welche 10 bis 20 Gew.-% Kupfer(II)oxid, 1 bis 5 Gew.-% Bismutoxid und 75 bis 89 Gew.-% Siliciumdioxid umfasst.

6. Verfahren nach einem der vorhergehenden Ansprüche, **dadurch gekennzeichnet, dass** man als katalytische Masse einen zuvor verwendeten, desaktivierten Katalysator einsetzt.

7. Verfahren nach einem der vorhergehenden Ansprüche, **dadurch gekennzeichnet, dass** man ein Alkin der Formel I einsetzt, worin R¹ für Alkyl oder Hydroxyalkyl steht.

8. Verfahren nach einem der vorhergehenden Ansprüche, **dadurch gekennzeichnet, dass** man ein Alkin der Formel I einsetzt, worin R² für ein Wasserstoffatom oder Alkyl steht.

9. Verfahren nach einem der vorhergehenden Ansprüche, **dadurch gekennzeichnet, dass** man eine Carbonylverbindung der Formel II einsetzt, worin R³ und R⁴ unabhängig voneinander für ein Wasserstoffatom oder Alkyl stehen, bevorzugt R³ und R⁴ beide für ein Wasserstoffatom stehen.

10. Katalysator, erhältlich durch ein Verfahren nach einem der Ansprüche 1 bis 9.

11. Verfahren zur Herstellung von Alkinolen der allgemeinen Formel III 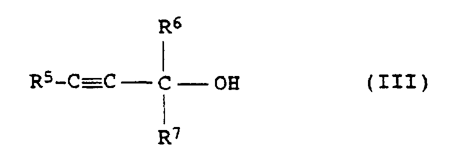 worin
R⁵ für ein Wasserstoffatom, Alkyl, Halogenalkyl, Cycloalkyl, Aryl, Alkoxy, Alkoxyalkyl oder einen -C(R⁶R⁷)OH-Substituenten steht,
R⁶ und R⁷ unabhängig voneinander für ein Wasserstoffatom, Alkyl, Halogenalkyl, Cycloalkyl oder Aryl stehen,
wobei man ein Gemisch aus einem Alkin der allgemeinen Formel IV
R⁵-C≡C-H (IV)
worin
R⁵ die oben angegebenen Bedeutungen besitzt, und
einer Carbonylverbindung der allgemeinen Formel V 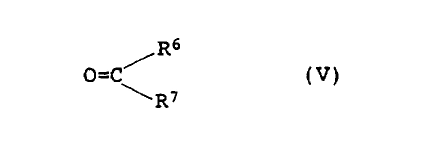 worin
R⁶ und R⁷ die oben angegebenen Bedeutungen besitzen,
umsetzt, **dadurch gekennzeichnet, dass** die Umsetzung in Gegenwart eines Katalysators nach Anspruch 10 erfolgt.

12. Verfahren zur Herstellung von Aminoalkinen der allgemeinen Formel VI 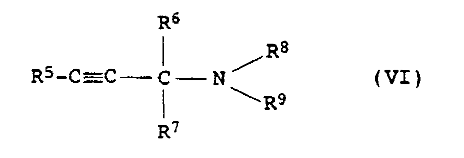 worin
R⁵, R⁶ und R⁷ die oben angegebenen Bedeutungen besitzen,
R⁸ und R⁹ unabhängig voneinander für ein Wasserstoffatom, Alkyl, Halogenalkyl, Cycloalkyl, Aryl oder Hydroxyalkyl stehen, oder
R⁸ und R⁹ zusammen mit dem Stickstoffatom, an das sie gebunden sind, einen 5- oder 6-gliedrigen, heterocyclischen Ring bilden;
wobei man ein Gemisch aus einem Alkin der allgemeinen Formel IV
R⁵-C≡C-H (IV)
worin
R⁵ die oben angegebenen Bedeutungen besitzt,
einer Carbonylverbindung der allgemeinen Formel V 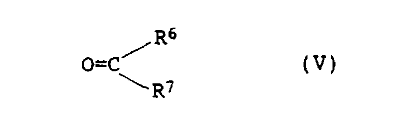 worin
R⁶ und R⁷ die oben angegebenen Bedeutungen besitzen, und
einem Amin der allgemeinen Formel VII 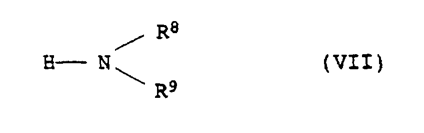 worin
R⁸ und R⁹ die oben angegebenen Bedeutungen besitzen,
umsetzt, **dadurch gekennzeichnet, dass** die Umsetzung in Gegenwart eines Katalysators nach Anspruch 10 erfolgt.

## Claims

1. A process for preparing a catalyst by activating a catalytic composition which comprises
a) at least one metal of group IB or IIB or a compound thereof,
b) where appropriate a carrier
which comprises treating the composition with an alkyne of the general formula I
R¹-C≡C-R² (I)
in which
R¹ is alkyl, cycloalkyl, aryl, hydroxyalkyl, haloalkyl, alkoxy or alkoxyalkyl,
R² is a hydrogen atom, alkyl, cycloalkyl or aryl,
and a carbonyl compound of the general formula II 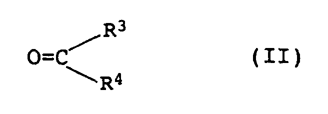 in which
R³ and R⁴ are, independently of one another, a hydrogen atom, alkyl, haloalkyl, cycloalkyl or aryl.

2. A process as claimed in claim 1, wherein the catalytic composition is treated under a pressure of up to 3 bar, preferably up to 2 bar, particularly preferably under atmospheric pressure.

3. A process as claimed in either of the preceding claims, wherein copper or a copper compound, preferably selected from copper oxide and malachite, is employed as component a).

4. A process as claimed in any of the preceding claims, wherein the catalytic composition also contains bismuth or a bismuth compound which is preferably selected from Bi₂O₃, (BiO)₂CO₃, Bi(NO₃)₃ and BiO(NO₃).

5. A process as claimed in any of the preceding claims, wherein a catalytic composition which comprises 10 to 20% by weight copper(II) oxide, 1 to 5% by weight bismuth oxide and 75 to 89% by weight silica is employed.

6. A process as claimed in any of the preceding claims, wherein a previously used, deactivated catalyst is employed as catalytic composition.

7. A process as claimed in any of the preceding claims, wherein an alkyne of the formula I in which R¹ is alkyl or hydroxyalkyl is employed.

8. A process as claimed in any of the preceding claims, wherein an alkyne of the formula I in which R² is a hydrogen atom or alkyl is employed.

9. A process as claimed in any of the preceding claims, wherein a carbonyl compound of the formula II in which R³ and R⁴ are, independently of one another, a hydrogen atom or alkyl, and preferably R³ and R⁴ are both a hydrogen atom, is employed.

10. A catalyst obtainable by a process as claimed in any of claims 1 to 9.

11. A process for preparing alkynols of the general formula III 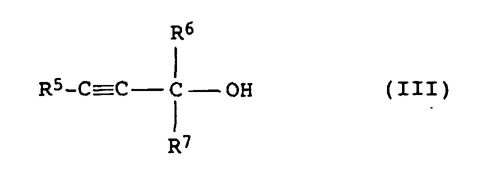 in which
R⁵ is a hydrogen atom, alkyl, haloalkyl, cycloalkyl, aryl, alkoxy, alkoxyalkyl or a -C(R⁶R⁷)OH substituent,
R⁶ and R⁷ are, independently of one another, a hydrogen atom, alkyl, haloalkyl, cycloalkyl or aryl,
by reacting a mixture of an alkyne of the general formula IV
R⁵-C≡C-H (IV)
in which
R⁵ has the meanings stated above, and
a carbonyl compound of the general formula V 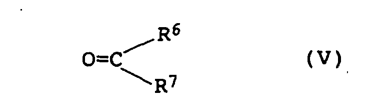 in which
R⁶ and R⁷ have the meanings stated above,
wherein the reaction takes place in the presence of a catalyst as claimed in claim 10.

12. A process for preparing aminoalkynes of the general formula VI 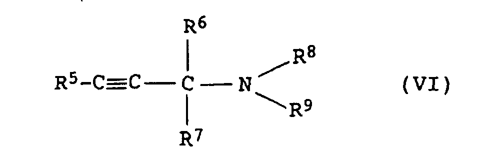 in which
R⁵, R⁶ and R⁷ have the meanings stated above,
R⁸ and R⁹ are, independently of one another, a hydrogen atom, alkyl, haloalkyl, cycloalkyl, aryl or hydroxyalkyl, or
R⁸ and R⁹ form, together with the nitrogen atom to which they are bonded, a 5- or 6-membered heterocyclic ring;
by reacting a mixture of an alkyne of the general formula IV
R⁵-C≡C-H (IV)
in which
R⁵ has the meanings stated above,
a carbonyl compound of the general formula V 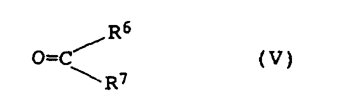 in which
R⁶ and R⁷ have the meanings stated above, and
an amine of the general formula VII 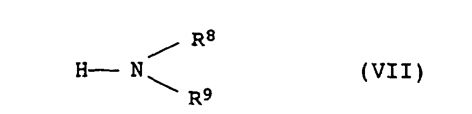 in which
R⁸ and R⁹ have the meanings stated above,
wherein the reaction takes place in the presence of a catalyst as claimed in claim 10.

## Revendications

1. Procédé de préparation d'un catalyseur par activation d'une masse catalytique qui contient
a) au moins un métal du premier ou du deuxième groupe secondaire, ou un composé de ce dernier,
b) éventuellement un support,
**caractérisé en ce qu'**on traite la masse avec un alcyne de formule générale I
R¹-C≡C-R² (I)
dans laquelle
R¹ est un groupe alkyle, cycloalkyle, aryle, hydroxyalkyle, halogènalkyle, alcoxy ou alcoxyalkyle,
R² est un atome d'hydrogène ou un groupe alkyle, cycloalkyle ou aryle, et un groupe carbonyle de formule générale II 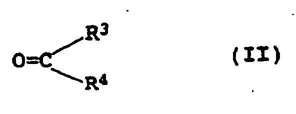 dans laquelle R³ et R⁴ représentent chacun indépendamment de l'autre un atome d'hydrogène ou un groupe alkyle, halogènalkyle, cycloalkyle ou aryle.

2. Procédé selon la revendication 1, **caractérisé en ce que** le traitement de la masse catalytique est réalisé sous une pression allant jusqu'à 3 bar, de préférence jusqu'à 2 bar, plus particulièrement sous la pression ambiante.

3. Procédé selon l'une des revendications précédentes, **caractérisé en ce qu'**on utilise en tant que composant a) du cuivre ou un composé du cuivre, de préférence choisi parmi l'oxyde de cuivre et la malachite.

4. Procédé selon l'une des revendications précédentes, **caractérisé en ce que** la masse catalytique contient aussi du bismuth ou un composé du bismuth, qui de préférence est choisi parmi Bi₂O₃, (BiO)₂CO₃, Bi(NO₃)₃ et BiO(NO₃).

5. Procédé selon l'une des revendications précédentes, **caractérisé en ce qu'**on utilise une masse catalytique qui contient de 10 à 20 % en poids d'oxyde de cuivre(II), de 1 à 5 % en poids d'oxyde de bismuth et de 75 à 89 % en poids de dioxyde de silicium.

6. Procédé selon l'une des revendications précédentes, **caractérisé en ce qu'**on utilise en tant que masse catalytique un catalyseur désactivé, déjà utilisé.

7. Procédé selon l'une des revendications précédentes, **caractérisé en ce qu'**on utilise un alcyne de formule I dans laquelle R¹ est un groupe alkyle ou hydroxyalkyle.

8. Procédé selon l'une des revendications précédentes, **caractérisé en ce qu'**on utilise un alcyne de formule I dans laquelle R² est un atome d'hydrogène ou un groupe alkyle.

9. Procédé selon l'une des revendications précédentes, **caractérisé en ce qu'**on utilise un composé carbonyle de formule II dans laquelle les radicaux R³ et R⁴ représentent chacun indépendamment de l'autre un atome d'hydrogène ou un groupe alkyle, R³ et R⁴ représentant de préférence chacun un atome d'hydrogène.

10. Catalyseur pouvant être obtenu par un procédé selon l'une des revendications 1 à 9.

11. Procédé de préparation d'alcynols de formule générale III 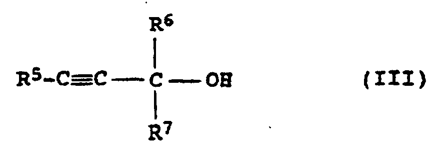 dans laquelle
R⁵ est un atome d'hydrogène ou un groupe alkyle, halogènalkyle, cycloalkyle, aryle, alcoxy, alcoxyalkyle, ou un substituant -C(R⁶R⁷)OH,
R⁶ et R⁷ représentent chacun indépendamment de l'autre un atome d'hydrogène ou un groupe alkyle, halogènalkyle, cycloalkyle ou aryle,
procédé dans lequel on fait réagir un mélange d'un alcyne de formule générale IV
R⁵-C≡C-H (IV)
dans laquelle R⁵ a les significations données ci-dessus,
et d'un composé carbonyle de formule générale V 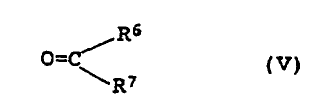 dans laquelle R⁶ et R⁷ ont les significations données ci-dessus,
**caractérisé en ce que** la réaction a lieu en présence d'un catalyseur selon la revendication 10.

12. Procédé de préparation d'aminoalcynes de formule générale VI 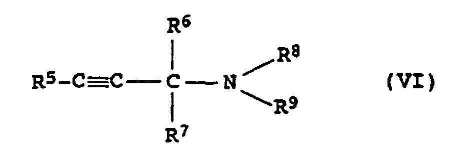 dans laquelle
R⁵, R⁶ et R⁷ ont les significations données ci-dessus,
R⁸ et R⁹ représentent chacun indépendamment de l'autre un atome d'hydrogène ou un groupe alkyle, halogènalkyle, cycloalkyle, aryle ou hydroxyalkyle, ou
R⁸ et R⁹, avec l'atome d'azote auquel ils sont liés, forment un noyau hétérocyclique à 5 ou 6 chaînons,
dans lequel on fait réagir un mélange d'un alcyne de formule générale IV
R⁵-C≡C-H (IV)
dans laquelle R⁵ a les significations données ci-dessus,
d'un composé carbonyle de formule générale V 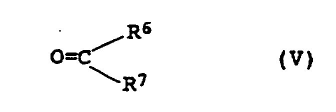 dans laquelle R⁶ et R⁷ ont les significations données ci-dessus, et
d'une amine de formule générale VII 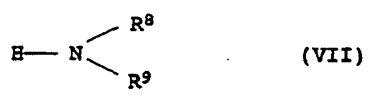 dans laquelle R⁸ et R⁹ ont les significations données ci-dessus,
**caractérisé en ce que** la réaction a lieu en présence d'un catalyseur selon la revendication 10.
